# EUROPÄISCHE PATENTSCHRIFT

(11) **EP 1 263 451 B1**
(45) Veröffentlichungstag und Bekanntmachung des Hinweises auf die Patenterteilung: **30.11.2005**
(21) Anmeldenummer: 00987324.1
(22) Anmeldetag: 30.11.2000
(51) Int. Cl.: A61K 35/78, A61K 9/52

(54) **HARTGELATINEKAPSELN ENTHALTEND RETARDIERTE PFLANZENEXTRAKTE UND VERFAHREN ZUR HERSTELLUNG DERSELBEN**
HARD GELATINE CAPSULES CONTAINING SUSTAINED-RELEASE PLANT EXTRACTS AND METHOD FOR PRODUCTION THEREOF
CAPSULES EN GELATINE DURE CONTENANT DES EXTRAITS DE PLANTE A LIBERATION CONTROLEE ET PROCEDE DE PRODUCTION DE CES CAPSULES

(30) Priorität: 15.03.2000 DE 10012569
(43) Veröffentlichungstag der Anmeldung: 11.12.2002
(73) Patentinhaber: Finzelberg GmbH & Co. KG, 56626 Andernach (DE)
(72) Erfinder: GAEDCKE, Frauke, 56323 Waldesch (DE); BERKULIN, Willi, 56237 Alsbach (DE)
(74) Vertreter: Werner, Hans-Karsten
(86) Internationale Anmeldenummer: PCT/EP2000/011987
(87) Internationale Veröffentlichungsnummer: WO 2001/068111

(56) Entgegenhaltungen:
- EP-A- 0 900 563
- WO-A-00/20017
- WO-A-00/69414
- WO-A-00/74656
- DE-A- 3 801 025
- DATABASE WPI Week 18923 Derwent Publications Ltd., London, GB; AN 1989-167839 XP002165366 & HU 47 858 A (KERBOLT)

## Beschreibung

Gegenstand der vorliegenden Erfindung sind Hartgelatinekapseln enthaltend retardierte Pflanzenextrakte sowie Verfahren zur Herstellung derselben.

Hartgelatinekapseln mit retardierten Wirkstoffen werden nach den Stand der Technik hergestellt, in dem der Wirkstoff zusammen mit retardierenden Wirkstoffen, insbesondere Fetten, Wachsen oder Alginaten in einem Wirbelschichtgranulator zu Pellets verarbeitet werden. Dieses Herstellungsverfahren ist sehr aufwendig und damit sehr kostenintensiv. Pflanzenextrakte sind häufig empfindlich gegen Wasser und Temperatur, so dass es oftmals kaum möglich ist, brauchbare retardierte Pellets herzustellen, die für Kapselfüllungen geeignet sind.

Die Erfindung hat sich die Aufgabe gestellt, Hartgelatinekapseln enthaltend retardierte Pflanzenextrakte zur Verfügung zu stellen, die sicher, einfach und kostengünstig hergestellt werden können und dabei gewährleisten, dass die Pflanzenextrakte nicht durch Wasser und Temperatur geschädigt werden.

Diese Aufgabe wird erfindungsgemäß dadurch gelöst, dass die Hartgelatinekapseln eine rieselfähige Füllung enthalten aus mit Filmmitteln überzogenen Kompaktaten der Pflanzenextrakte oder von Gemischen der Pflanzenextrakte mit Hilfsstoffen.

Die Herstellung dieser Hartgelatinekapseln erfolgt dadurch, dass die Pflanzenextrakte
a) unverdünnt oder mit Hilfsstoffen vermischt zu Schülpen verpresst,
b) die Schülpen mechanisch zerkleinert werden,
c) von den zerkleinerten Schülpen Überkorn und Unterkorn abgetrennt,
d) die gewünschte mittlere Fraktion mit filmbildenden Mitteln überzogen und
e) in Hartgelatinekapseln abgefüllt wird.

Sofern die Pflanzenextrakte nicht unverdünnt zum Einsatz kommen können, können sie zuvor mit Hilfsstoffen vermischt werden, die im allgemeinen auch das Kompaktieren zu Schülpen erleichtern. Typische und bevorzugte Hilfsstoffe sind Lactose, Saccharose, Calcium Carbonat, Polyethylenglykole, Calciumhydrogenphosphat, Cellactose, mikrokristalline Celllulose, Cellulose-Derivate, Crosscarmellose und Gemische derselben.

Die Korngrößenverteilung der mit Filmmitteln überzogenen Kompaktate liegt im allgemeinen zwischen 0,2 bis 1,5 mm, vorzugsweise 0,3 bis 1,25 mm. Derartige mit filmbildenden Mittel überzogene Kompaktate sind gut rieselfähig und lassen sich daher ohne Schwierigkeiten in Hartgelatinekapseln abfüllen. Sowohl durch das Kompaktieren wie durch das Überziehen mit filmbildenden Mitteln erfolgt die geforderte Retardierung der Freisetzung nach der Applikation.

Die gewünschte Korngrößenverteilung ist am einfachsten zu erreichen durch Durchreiben durch ein erstes gröberes Sieb, wobei die Maschenweite die Komgrößenobergrenze bestimmt und durch Schütteln auf einem zweiten engmaschigen Sieb, wobei die Maschenweite die Korngrößenuntergrenze bestimmt wird.

Es ist aber durchaus möglich, auch diese gewünschte mittlere Fraktion durch Windsichtung von dem zunächst zerkleinerten Gemisch der verschiedenen Komgrößen abzutrennen.

Kompaktierung, Zerkleinerung und Abtrennung der gewünschten mittleren Korngrößenfraktionen kann beispielsweise erfolgen in Walzenpressen mit nachgeschaltetem Mahlsystem und nachgeschalteter Siebmaschine. Derartige Maschinen werden beispielsweise von der Gerteis Maschinen + Processengineering AG unter der Bezeichnung GMP-Polygran Walzenpresse zur Verfügung gestellt.

In derartigen Vorrichtungen wird dann der unverdünnte, pulverförmige Pflanzenextrakt oder das Gemisch mit Hilfsstoffen von einem Silo aus zudosiert, wobei gleichzeitig vorentlüftet wird. Durch die Pressrollen werden Schülpen hergestellt, welche beispielweise durch oszilierenden Granulatoren gebrochen und durch ein Sieb gearbeitet werden, so dass die gewünschte Korngröße entsteht. Oberkorn wird gegebenenfalls erneut der Zerkleinerungsstufe zugeführt, Unterkorn wird gewünschtenfalls dem noch pulverförmigen Ausgangsmaterial zugeführt.

Als filmbildende Mittel kommen die in der pharmazeutischen Industrie üblichen filmbildenden Mittel in Frage, wie Zein, Schellack oder die verschiedenen Eudragit®-Lösungen. Insbesondere aus den verschiedenen Eudragit®-Lösungen können mehr oder weniger retardierende Überzüge hergestellt werden, so dass die Retardierung der Hartgelatinekapseln insgesamt einstellbar ist.

Als Pflanzenextrakte, die retardiert in Hartgelatinekapseln abgefüllt werden sollen, kommen praktisch alle Pflanzenextrakte in Frage, die unretardiert zu rasch reagieren und nicht langsam genug freigesetzt werden. Beispiele hierfür sind Johanniskraut, Echinacea, Baldrian und Knoblauch.

## Patentansprüche

1. Hartgelatinekapseln enthaltend retardierte Pflanzenextrakte, **kennzeichnet durch** eine rieselfähige Füllung bestehend aus
- mit Filmmitteln überzogenen Kompaktaten der Pflanzenextrakte
oder
- mit Filmmitteln überzogene Kompaktaten von Gemischen der Pflanzenextrakte mit Hilfsstoffen.

2. Hartgelatinekapseln gemäß Anspruch 1, **dadurch gekennzeichnet, dass** die Hilfsstoffe Lactose, Saccharose, Calcium Carbonat, Polyethylenglykole, Calciumhydrogenphosphat, Cellactose, mikrokristalline Celllulose, Cellulose-Derivate, Crosscarmellose und Gemische derselben sind.

3. Hartgelatinekapseln gemäß Anspruch 1 oder 2, **dadurch gekennzeichnet, dass** die mit Filmmitteln überzogenen Kompaktate eine Korngrößenverteilung von 0,2 bis 1,5 mm, vorzugsweise 0,3 bis 1,25 mm aufweisen.

4. Hartgelatinekapseln gemäß einem der Ansprüche 1 bis 3, **dadurch gekennzeichnet, dass** die filmbildenden Mittel Zein, Schellack oder Eudragit®-Lösungen sind.

5. Verfahren zur Herstellung von Hartgelatinekapseln mit retardierten Pflanzenextrakten, **dadurch gekennzeichnet, dass** die Pflanzenextrakte
a) unverdünnt oder mit Hilfsstoffen vermischt zu Schülpen verpresst,
b) die Schülpen mechanisch zerkleinert werden,
c) von den zerkleinerten Schülpen Überkorn und Unterkorn abgetrennt,
d) die gewünschte mittlere Fraktion mit filmbildenden Mitteln überzogen und
e) in Hartgelatinekapseln abgefüllt wird.

6. Verfahren gemäß Anspruch 5, **dadurch gekennzeichnet, dass** als Hilfsstoffe verwendet werden Lactose, Saccharose, Calcium Carbonat, Polyethylenglykole, Calciumhydrogenphosphat, Cellactose, mikrokristalline Celllulose, Cellulose-Derivate, Crosscarmellose und Gemische derselben.

7. Verfahren gemäß Anspruch 5 oder 6, **dadurch gekennzeichnet, dass** die Schritte b) und c) erfolgen durch Durchreiben durch ein erstes gröberes Sieb, wobei die Maschenweite die Komgrößenobergrenze bestimmt und durch Schütteln auf einem zweiten engmaschigen Sieb, wobei die Maschenweite die Korngrößenuntergrenze bestimmt wird.

8. Verfahren gemäß Anspruch 5 oder 6, **dadurch gekennzeichnet, dass** die Schritte b) und c) durch Windsichtung erfolgt.

9. Verfahren gemäß einem der Ansprüche 5 bis 8, **dadurch gekennzeichnet, dass** die Korngrößenverteilung der mittleren Fraktion im Bereich von 0,2 bis 1,5 mm, vorzugsweise 0,3 bis 1,25 mm liegt.

10. Verfahren gemäß einem der Ansprüche 5 bis 9, **dadurch gekennzeichnet, dass** als filmbildende Mittel Zein, Schellack oder Eudragit®-Lösungen verwendet werden.

## Claims

1. Hard gelatin capsules comprising sustained-release plant extracts, **characterized by** a free-flowing filling consisting of
- compactates of the plant extracts coated with film agents; or
- compactates of mixtures of the plant extracts with auxiliary agents coated with film agents.

2. The hard gelatin capsules according to claim 1, **characterized in that** said auxiliary agents are lactose, sucrose, calcium carbonate, polyethylene glycols, calcium hydrogenphosphate, cellactose, microcrystalline cellulose, cellulose derivatives, cross-carmellose or mixtures thereof.

3. The hard gelatin capsules according to claim 1 or 2, **characterized in that** said compactates coated with film agents have a grain size distribution of from 0.2 to 1.5 mm, preferably from 0.3 to 1.25 mm.

4. The hard gelatin capsules according to any of claims 1 to 3, **characterized in that** the film-forming agents are zein, shellac or Eudragit® solutions.

5. A method for the preparation of the hard gelatin capsules with sustained-release plant extracts, **characterized in that**
a) the plant extracts, which are undiluted or mixed with auxiliary agents, are pressed to ribbons;
b) the ribbons are mechanically comminuted;
c) the oversize and undersize are separated from the comminuted ribbons;
d) the desired medium fraction is coated with film-forming agents; and
e) filled into hard gelatin capsules.

6. The method according to claim 5, **characterized in that** lactose, sucrose, calcium carbonate, polyethylene glycols, calcium hydrogenphosphate, cellactose, microcrystalline cellulose, cellulose derivatives, cross-carmellose or mixtures thereof are used as auxiliary agents.

7. The method according to claim 5 or 6, **characterized in that** steps b) and c) are performed by passing through a first coarser sieve, the mesh size determining the grain size upper limit, and vibrating on a second, narrow-meshed sieve, the mesh size now determining the grain size lower limit.

8. The method according to claim 5 or 6, **characterized in that** steps b) and c) are performed by air classification.

9. The method according to any of claims 5 to 8, **characterized in that** the grain size distribution of said medium fraction ranges from 0.2 to 1.5 mm, preferably from 0.3 to 1.25 mm.

10. The method according to any of claims 5 to 9, **characterized in that** zein, shellac or Eudragit® solutions are used as the film-forming agents.

## Revendications

1. Capsules en gélatine dure contenant des extraits de plantes à libération contrôlée, **caractérisées par** une charge coulante constituée de :
- masses compactées d'extraits de plantes revêtues d'agents filmogènes
ou
- masses compactées de mélanges d'extraits de plantes d'auxiliaires, revêtues d'agents filmogènes.

2. Capsules en gélatine dure selon la revendication 1, **caractérisée en ce que** les auxiliaires sont le lactose, le saccharose, le carbonate de calcium, les polyéthylène glycols, l'hydrogénophosphate de calcium, le cellactose, la cellulose microcristalline, les dérivés de cellulose, la crosscarmellose et leurs mélanges.

3. Capsules en gélatine dure selon la revendication 1 ou 2, **caractérisée en ce que** les masses compactées revêtues d'agents filmogènes présentent une distribution granulométrique de 0,2 à 1,5 mm, de préférence de 0,3 à 1,25 mm.

4. Capsules en gélatine dure selon l'une des revendications 1 à 3, **caractérisée en ce que** les agents filmogènes sont des solutions de zéine, shellac ou Eudragit®.

5. Procédé de fabrication de capsules en gélatine dure avec des extraits de plantes à libération contrôlée, **caractérisé en ce que** :
a) les extraits de plantes sont comprimés en gales à l'état non dilué ou mélangé avec des auxiliaires,
b) les gales sont concassées mécaniquement,
c) on sépare les gros grains et les petits grains parmi les gales concassées,
d) la fraction médiane voulue est revêtue d'agents filmogènes et
e) chargée dans des capsules en gélatine dure.

6. Procédé selon la revendication 5, **caractérisé en ce que** l'on utilise comme auxiliaires le lactose, le saccharose, le carbonate de calcium, les polyéthylène glycols, l'hydrogénophosphate de calcium, le cellactose, la cellulose microcristalline, les dérivés de cellulose, la crosscarmellose et leurs mélanges.

7. Procédé selon la revendication 5 ou 6, **caractérisé en ce que** les étapes b) et c) s'effectuent par frottement à travers un premier tamis plus gros, où la largeur des mailles détermine la limite supérieure granulométrique, et par agitation sur un deuxième tamis à maille étroite, la largeur des mailles déterminant la limite inférieure granulométrique.

8. Procédé selon la revendication 5 ou 6, **caractérisé en ce que** les étapes b) et c) s'effectuent par criblage à l'air.

9. Procédé selon l'une des revendications 5 à 8, **caractérisé en ce que** la distribution granulométrique de la fraction médiane se situe dans la plage de 0,2 à 1,5 mm, de préférence de 0,3 à 1,25 mm.

10. Procédé selon l'une des revendications 5 à 9, **caractérisé en ce que** l'on utilise à titre d'agents filmogènes des solutions de zéine, shellac ou Eudragit®.
